# EUROPEAN PATENT SPECIFICATION

(11) **EP 1 833 815 B1**
(45) Date of publication and mention of the grant of the patent: **15.09.2010**
(21) Application number: 05822030.2
(22) Date of filing: 30.12.2005
(51) Int. Cl.: C07D 401/04

(54) **A PROCESS FOR PREPARATION OF IMATINIB BASE**
VERFAHREN ZUR HERSTELLUNG VON IMATINIB-BASE
PROCEDE DE PREPARATION D'UNE BASE D'IMATINIBE

(30) Priority: 30.12.2004 PL 37201604; 19.08.2005 PL 37669105; 08.11.2005 PL 37798405
(43) Date of publication of application: 19.09.2007
(73) Proprietor: INSTYTUT FARMACEUTYCZNY, PL-01-793 Warszawa (PL)
(72) Inventor: SZCZEPEK, Wojciech, PL-01-493 Warszawa (PL); LUNIEWSKI, Wojciech, PL-00-355 Warszawa (PL); KACZMAREK, Lukasz, PL-02-904 Warszawa (PL); ZAGRODZKI, Bogdan, PL-00-170 Warszawa (PL); SAMSON-LAZINSKA, Dorota, PL-04-937 Warszawa (PL); SZELEJEWSKI, Wieslaw, PL-03-980 Warsyawa (PL); SKARZYNSKI, Maciej, PL-03-914 Warszawa (PL)
(74) Representative: Krzywdzinska, Ewa
(86) International application number: PCT/PL2005/000088
(87) International publication number: WO 2006/071130

(56) References cited:
- EP-A- 0 564 409
- WO-A-2004/099186
- WO-A-2004/108699
- WO-A-2006/021458
- ZIMMERMANN, J. ET AL.: "PHENYLAMINO-PYRIMIDINE (PAP) DERIVATIVES: A NEW CLASS OF POTENT ANDSELECTIVE INHIBITORS OF PROTEIN KINASE C (PKC)" ARCHIV DER PHARMAZIE, VCH VERLAGSGESELLSCHAFT MBH, WEINHEIM, DE, vol. 329, no. 7, July 1996 (1996-07), pages 371-376, XP000885618 ISSN: 0365-6233
- ZIMMERMANN, J. ET AL: "Phenylamino-pyrimidine (PAP) - derivatives: a new class of potent and highly selective PDGF-receptor autophosphorylation inhibitors" BIOORGANIC & MEDICINAL CHEMISTRY LETTERS, OXFORD, GB, vol. 6, no. 11, 4 June 1996 (1996-06-04), pages 1221-1226, XP004134858 ISSN: 0960-894X
- BECKER, H. G. O. ET AL.: "Organikum - Organisch-Chemisches Grundpraktikum, 15. Auflage" 1981, VEB DEUTSCHER VERLAG DER WISSENSCHAFTEN , BERLIN , XP002389624 Pages 645-649.
- SZAKÁCS, Z. ET AL.: "Acid-Base Profiling of Imatinib (Gleevec) and Its Fragments" J. MED. CHEM., vol. 48, 2005, pages 249-255, XP002389622 cited in the application

## Description

### Field of the invention

The invention relates to an improved process for preparation of imatinib base and its pharmaceutically acceptable acid addition salts. The invention comprises also processes for preparing intermediates in the imatinib synthesis.

### Background of the invention

Imatinib, 4-[(4-methyl-1-piperazinel)methyl]-N-[4-methyl-3-[[4-(3-pyridinyl)-2-pyrimidynyl]amino]-phenyl]benzamide, a selective inhibitor of tyrosine kinase, disclosed in the European patent application EP 0564409 A1, is represented by the structural formula:

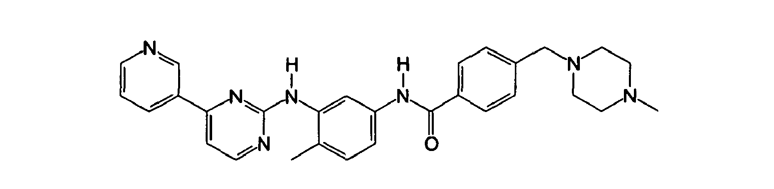

The addition salt of imatinib with methanesulfonic acid is the active ingredient of oral compositions for the treatment of patients with Philadelphia chromosome positive chronic meyloid leukemia and with Kit-positive unresectable and/or metastatic malignant gastrointestinal stromal tumours.

Three basic synthetic pathways for preparation of imatinib base are known in the prior art.

Scheme 1 illustrates the method disclosed in the European patent application EP 0564409 A1 and in the PCT patent application publication WO 2004/074502, that comprises a condensation of two moieties: N-(5-amino-2-methylphenyl)-4-(3-pyridinyl)-2-pyrimidineamine and 4-(4-methylpiperazinelmethyl)benzoyl chloride (Method A).

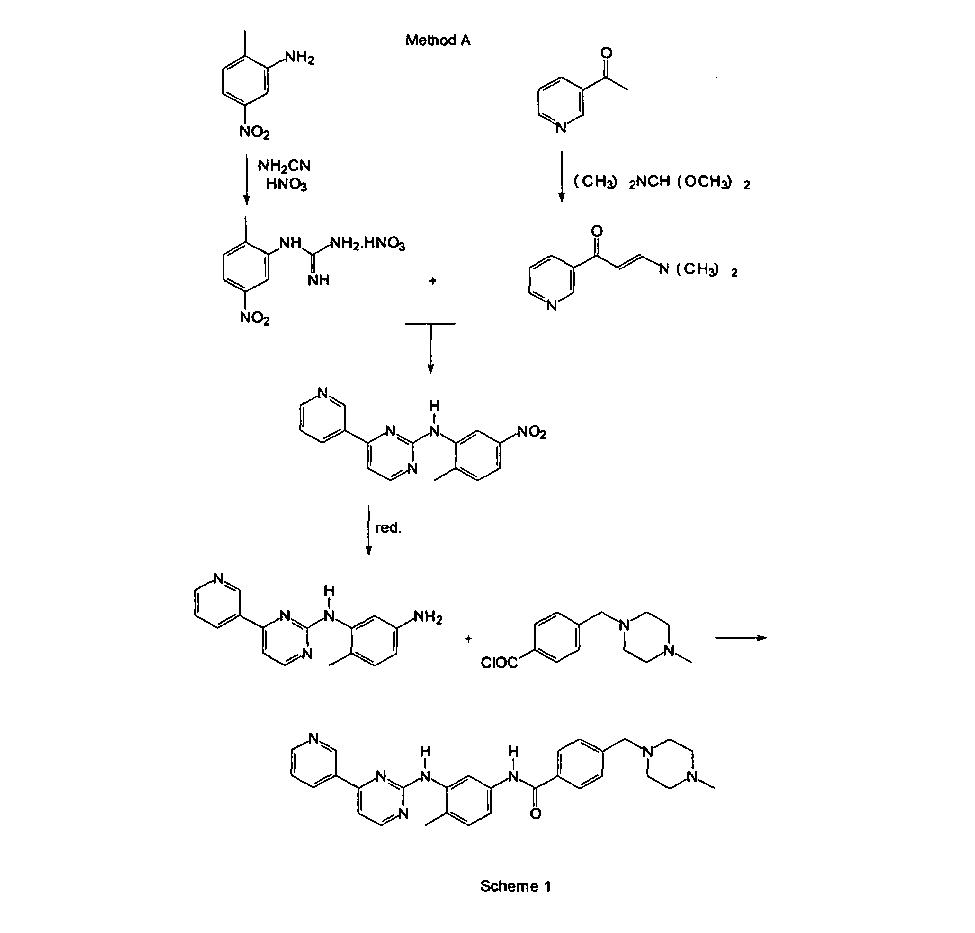

Scheme 2 illustrates the process for preparing 4-[(4-methyl-1-piperazinel)methyl]-N-[4-methyl-3-[[4-(3-pyridinyl)-2-pyrimidinyl]amino]-phenyl]benzamide by condensing synthons of the molecule in another order, described in the publication WO 03/066613 (Method B). In Method B, (3-pyridinyl)-2-pyrimidineamine or a precursor thereof is reacted with 4-methylphenyl-(4-methylpiperazin-1-ylmethyl)-benzamide.

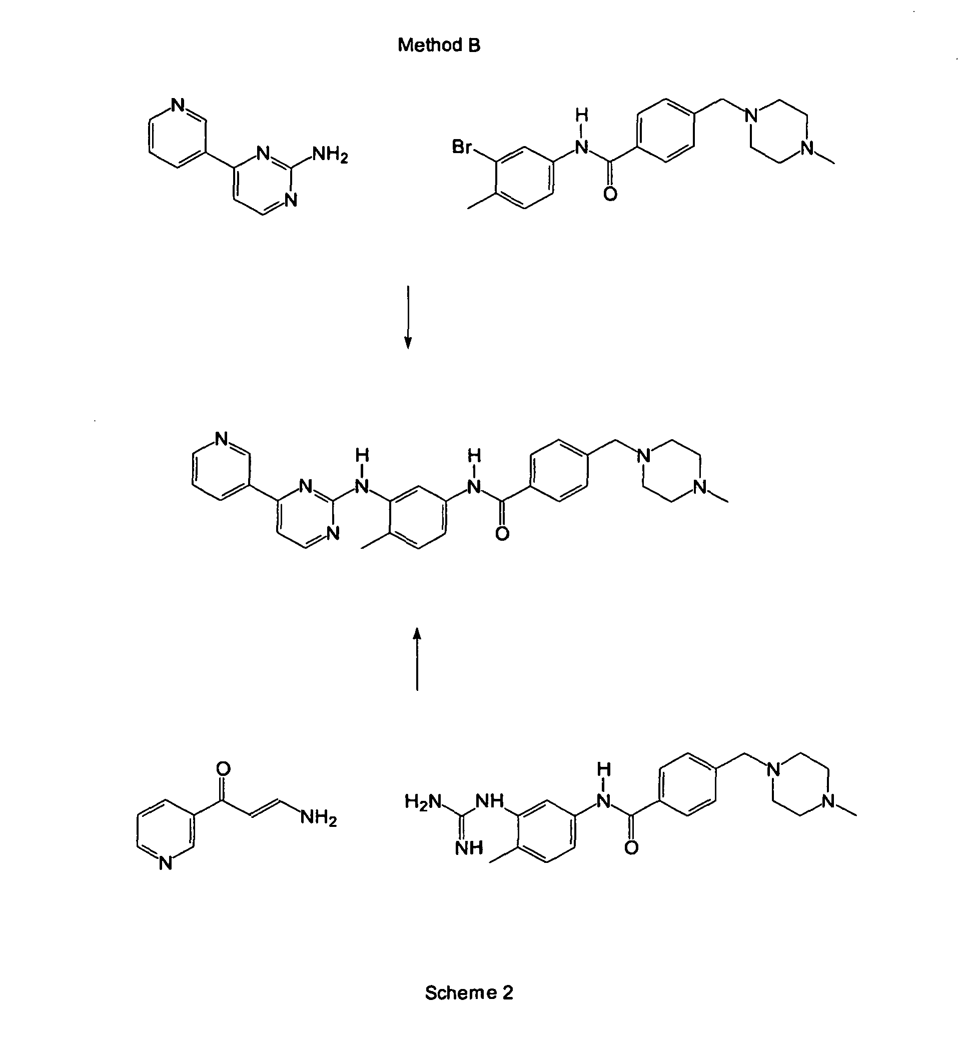

Scheme 3 presents a method of imatinib synthesis described in the publication WO 2004/108699 and in J. Med. Chem. 2005, 48(1), 249-255 (Method C). In that method, at first, 4-methyl-N-3-(4-pyridin-3-yl-pyrimidin-2-yl)benzene-1,3-diamine is condensed with 4-chloromethylbenzoyl chloride and then the resulting 4-chloromethyl-N-{4-methyl-3-[(4-pyridin-3-yl)-pyrimidin-2-ylamino]-phenyl}-benzamide is reacted with N-methylpiperazine.

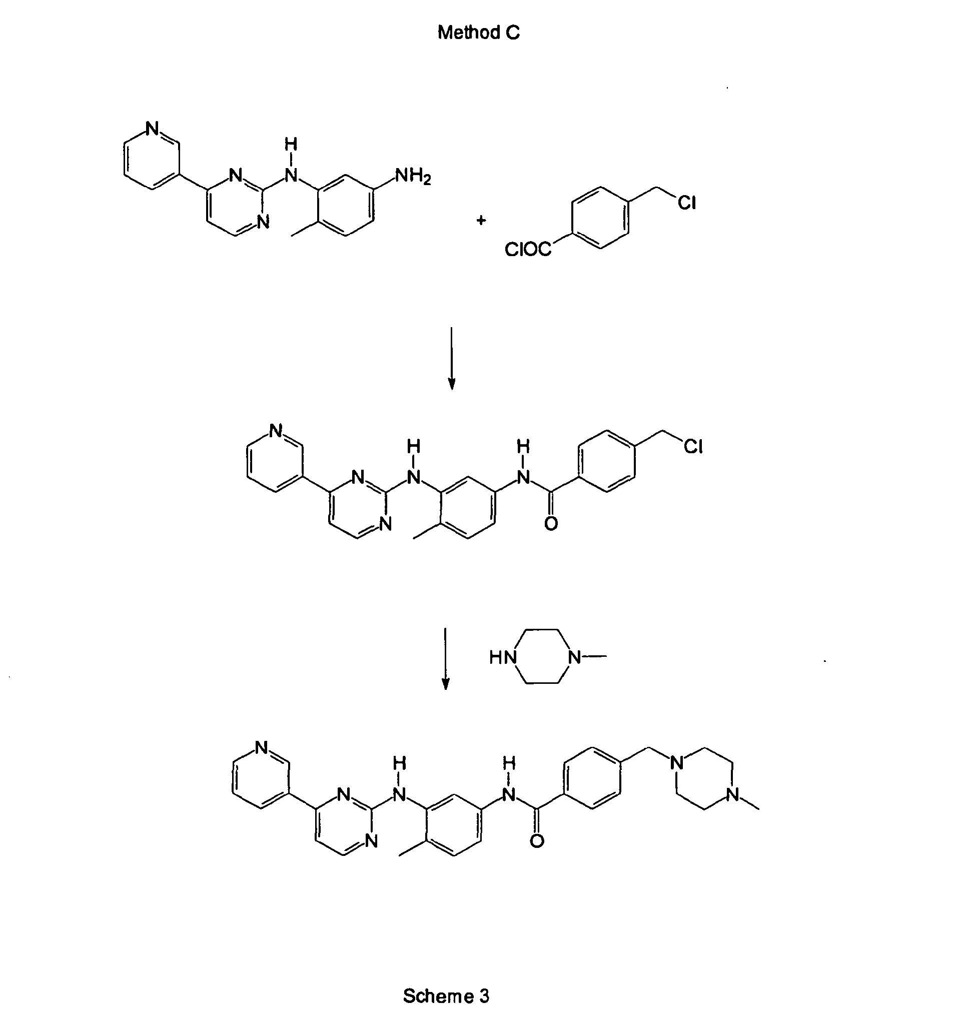

The methods known from the art are characterized by some inconveniences which make them troublesome and difficult to scale up.

The first key intermediate in the synthesis of imatinib by Methods A and C is N-(5-nitro-2-methylphenyl)-4-(3-pyridinyl)-2-pyrimidineamine that, according to the disclosure of EP 0564409 A1 is obtained by condensation of 1-(2-methyl-5-nitrophenyl)guanidine with 3-(dimethylamino)-1-(3-pyridinyl)-prop-2-en-1-one.

According to the disclosure of the patents US 4,281,000 (corresponding to EP 0025819 B1) and US 4,374,988, one of the reagents, 3-(dimethylamino)-1-(3-pyridinyl)-prop-2-en-1-one, is obtained by refluxing a mixture of 3-acetylpyridine with a molar excess (1.09-4.7 equivalent) of N,N-dimethylformamide dimethyl acetal for 6-16 hours. The resulting product is isolated by concentrating the reaction mixture *in vacuo*, treating the residue with hexane and filtering the formed crystals. Usually, the crude product obtained as above has to be purified by direct recrystallization from a suitable solvent or by filtration of a solution of the crude product in methylene chloride through a layer of magnesium silicate followed by recrystallization. Alternatively, the purified product is isolated by concentrating the reaction mixture *in vacuo*, dissolving the residue in methylene chloride and filtering the obtained solution through a layer of magnesium silicate followed by recrystallization. Unfortunately, the compound is well soluble in organic solvents and in water and therefore, yield of the product obtained by recrystallization is poor (50-66%) as compared to that of the solidified crude product (85-89%). For example, after recrystallization of the crude product, the yield of 3-(dimethylamino)-1-(3-pyridinyl)-prop-2-en-1-one obtained according to US 4,281,000 in the reaction of 3-acetylpyridine with 1.09 equivalent of N,N-dimethylformamide dimethyl acetal (Example 1) is only 50%.

Another cause of poor yield of 3-(dimethylamino)-1-(3-pyridinyl)-prop-2-en-1-one could be incomplete conversion of 3-acetylpyridine.

According to EP 0233461 B1 and EP 0564409 A1 the other reagent, 1-(2-methyl-5-nitrophenyl)guanidine, can be obtained by heating 2-methyl-5-nitroaniline in the presence of an aqueous solution of cyanamide and, if necessary, an acid that would be a source of appropriate anion, for example, in the presence of concentrated nitric acid. The resulting product is isolated from the reaction mixture in the crystalline form.

In EP 0564409 A1, the reaction of 1-(2-methyl-5-nitrophenyl)guanidine with 3-(dimethylamino)-1-(3-pyridinyl)-prop-2-en-1-one is carried out by a long-lasting (6-48 hours) heating the reagents in a neutral solvent, in the presence of appropriate base, if necessary. The yield of N-(5-nitro-2-methylphenyl)-4-(3-pyridinyl)-2-pyrimidineamine, obtained according to a procedure disclosed in the experimental part of EP 0564409 did not exceed 75%.

Therefore, the prior art methods for preparing N-(5-nitro-2-methylphenyl)-4-(3-pyridinyl)-2-pyrimidineamine consist of many steps, are time-consuming and require purification of intermediates at subsequent steps of the synthesis which additionally decreases the total yield of the product.

Both methods, A and C, requires the reduction of the nitro compound, N-(5-nitro-2-methylphenyl)-4-(3-pyridinyl)-2-pyrimidineamine to N-(5-amino-2-methylphenyl)-4-(3-pyridinyl)-2-pyrimidineamine.

In EP 0564409 A1 reduction of a nitro group to an amino derivative in N-(substituted nitrophenyl)-4-(3-pyridinyl)-2-pyrimidineamines is carried out using hydrogen in the presence of palladium on a support [10% Pd(C)] and ethyl acetate as a solvent, at room temperature and under ambient pressure for 6.5 hours or in the presence of palladium catalyst [5% Pd(C)] using tetrahydrofuran as a solvent, at room temperature and under ambient for 21 hours. No yield of the product has been disclosed in any example.

The reduction of N-(5-nitro-2-methylphenyl)-4-(3-pyridinyl)-2-pyridineamine to N-(5-amino-2-methylphenyl)-4-(3-pyridinyl)-2-pyridineamine by a chemical method, described in the publications WO 2004/074502 and WO 2004/108699 is carried out using tin(II) chloride - SnCl₂. According to WO 2004/074502, the reaction with tin(II) chloride is carried out in tetrahydrofuran at 60°C for 4 hours or in a mixture of water and concentrated hydrochloric acid at 50°C for 2 hours. No yield of the product, obtained after recrystallization from ethyl acetate has been given in the provided examples. Only in the description of this application the authors have disclosed that the yield of the product was 65-70%. Hence, the yield was higher than that obtained in the reconstructed examples from EP 0564409 (in which, according to the authors of WO 2004/074502, the yields are approximately 40-45%), purity of the product was higher and consumption of organic solvents was lower. In WO 2004/108699, reduction with the use of tin(II) chloride was carried out in concentrated hydrochloric acid at temperature 0-5°C for 3-4 hours and then at 25-35°C for 1.5 hour to afford, after recrystallization from ethyl acetate, the product with the yield 60-64.5%.

A paper published in J. Med. Chem., 2005, 48(1), 249-255, describes a reduction of N-(5-nitro-2-methylphenyl)-4-(3-pyridinyl)-2-pyridineamine using SnCl₂.H₂O in concentrated hydrochloric acid at room temperature for 30 min., where the yield of the amine after recrystallization of the crude product from methylene chloride was 81%. The yield is higher compared to that reported in the publications WO 2004/074 502 and WO 2004/ 108 699.

According to the common knowledge, reduction of aromatic nitro compounds to corresponding amines can be accomplished:
- by using metals such as zinc (Zn), tin (Sn), iron (Fe), etc. in an acidic medium (hydrochloric acid), or by using tin (II) chloride in an acidic medium (hydrochloric acid) or alternatively, by using zinc (Zn) in an alkaline medium (sodium hydroxide);
- by catalytic hydrogenation; using hydrogen in the presence of catalysts such as Pd(C), Pt(C) and Ni(Raney), by using hydrazine in the presence of catalysts such as Pd(C), Pt, Ru and Ni(Raney) or by using formic acid (HCOOH) in the presence of Pd(C); and
- by using sulfur compounds such as, e.g., H₂S, NaHS, Na₂S, (NH₄)₂S, Na₂S₂O₄ or other as reducing agents

In most cases, when nitro compounds are being reduced to amines in an acidic medium and in cases of catalytic reduction in a neutral medium, the reaction proceeds practically quantitatively and no by-products are formed. However, a high cost of the catalysts and/or the necessity of using equipment suitable for operation under pressure of hydrogen are disadvantages of these methods.

On the other hand, various by-products, such as azoxy-, azo- or hydrazo-derivatives, can be formed in the case of a reduction in an alkaline medium that under these conditions do not undergo further reduction to amines. In the industrial scale production, it makes difficult or even precludes isolation of a pure amine. Disadvantages of a reduction using metals in an acidic medium are formation of considerable amounts of inorganic salts that are wastes hard to dispose of and problems associated with isolating the amine after alkalizing the reaction mixture.

In the next step of Method A, N-(5-amino-2-methylphenyl)-4-(3-pyridinyl)-2-pyridineamine is reacted with 4-(4-methylpiperazinemethyl)benzoyl chloride, wherein the latter one is used as dihydrochloride. The reaction requires large volume of an organic base, such as pyridine or triethylamine, that binds the evolving hydrogen chloride.

According to Example 21 of EP 0564409 B1, the reaction is carried out by a long-lasting heating of reagents in pyridine. No reaction yield is given in the description. One can assume that the yield is not high considering potential side reactions accompanying the major process. Moreover, the yield is additionally decreased due to the necessity of pyridine removal that is difficult and requires a laborious work-up in order to isolate and purify the final product.

A modification of the method A has been proposed in the publication of PCT application WO 2004/074502, wherein the condensation is carried out in a neutral organic solvent and, although 4-(4-methylpiperazinemethyl)benzoyl chloride is used as a di(hydrogen halide), no hydrogen chloride binding agent is used. A tri(hydrogen halide) salt of imatinib, preferably imatinib trihydrochloride in the form of a hydrate is obtained, that precipitates from the solution, therefore facilitating its isolation. The yield of imatinib trihydrochloride hydrate obtained in this step is a mere 53.5%. Before converting it to methanesulfonic acid addition salt, imatinib trihydrochloride obtained by this method requires an additional step, in which imatinib free base is liberated, further decreasing the total yield. The yield of converting trihydrochloride into imatinib base is not revealed in the description but the combined yield of two steps can not be considered as high.

In Method B, (3-pyridinyl)-2-pyrimideineamine or a precursor thereof is reacted with 4-methylphenyl-(4-methylpiperazin-1-ylmethyl)benzamide.

In one variation of Method B (Example 9 of WO 03/066613), a suspension of N-(3-guanidine-4-methylphenyl)-4-(4-methylpiperazin-1-ylmethyl)-benzamide is heated in n-butanol with a slight molar excess of 3-dimethylamino-1-pyridin-3-ylpropenone. The process requires high temperature and has to be carried out in an inert gas atmosphere. The product is isolated by distilling off dimethylamine with n-butanol, making up its amount in the reaction mixture during distillation and then precipitating the product using butyl acetate.

Another variation of Method B (Example 10 of WO 03/066613), comprises a reaction of N-(3-bromo-4-methylphenyl)-4-(4-methylpiperazin-1-ylmethyl)benzamide with 4-(3-pyridinyl)-2-pyrimidineamine in the presence of sodium *tert*-butoxide and a catalytic amount of Pd₂(dba)₃ - rac-BINAP. Despite a relatively high yield of the last step of condensing key synthons of the compound, the synthesis of imatinib by this method is difficult for scaling-up as it requires expensive catalysts, special equipment for sonication of the reaction mixture, as well as onerous purification of the product by "flash" chromatography on a silica gel and separating it from undesired isomers using reverse-phase preparative chromatography.

A variation of Method C, described in J.Med.Chem. 2005, 48(1), 249-255, is not of significant practical importance. It uses expensive analytical-grade reagents and the yields obtained in subsequent steps are rather poor. Namely, the yield of the reaction of 4-methyl-N-3-[(4-pyridin-3-yl)pyrimidin-2-yl]benzene-1,3-diamine with 4-chloromethylbenzoyl chloride in dimethylformamide is 61% after recrystallization of the product from ethyl acetate, and the yield of the reaction of 4-chloromethyl-N-{[4-methyl-3-(4-pyridin-3-yl)pyrimidin-2-ylamino]-phenyl}-benzamide with N-methylpiperazine, after recrystallizing the product from acetonitrile, is 68%.

Reassuming, the multi-step total synthesis of imatinib base is still requiring novel solutions that would decrease its consumption of time and labour. Accounting for the need of carrying out numerous unit operations resulting from a complex structure of the molecule, it is of particular importance to develop methods that would allow for increasing yields of particular steps, which in turn would favour an increase of the yield of the whole technology. Development of novel methods for accomplishing subsequent synthetic steps, suitable for scaling-up and more efficient economically, seems to be of particular importance.

### Short description of the invention

The invention provides a process for preparation of imatinib base and pharmaceutically acceptable acid addition salts thereof, comprising:
a) preparation of a 1-(2-methyl-5-nitrophenyl)guanidine nitrate by reacting 2-methyl-5-nitroaniline with cyanamide in the presence of concentrated hydrochloric acid and conversion of the resulting hydrochloride acid salt into a nitric acid salt;
b) independently, condensation of 3-acetylpyridine with N,N-dimethylformamide dimethyl acetal under conditions favouring removal of methanol formed in the reaction, to obtain crude 3-(dimethylamino)-1-(3-pyridinyl)-prop-2-en-1-one, which, without further purification, is
c) reacted directly with 1-(2-methyl-5-nitrophenyl)-guanidine nitrate obtained in step a);
d) reduction of N-(5-nitro-2-methylphenyl)-4-(3-pyridinyl)-2-pyridineamine obtained in the step (c) to N-(5-amino-2-methylphenyl)-4-(3-pyridinyl)-2-pyridineamine using hydrazine in the presence of Raney's nickel;
e) condensation of N-(5-nitro-2-methylphenyl)-4-(3-pyridinyl)-2-pyridineamine obtained in step d) with 4-chloromethylbenzoyl chloride in the presence of a base;
f) condensation of 4-chloromethyl-N-{[4-methyl-3-(4-pyridin-3-yl)pyrimidin-2-ylamino]-phenyl}-benzamide obtained in step e) with a molar excess of N-methylpiperazine;
g) dilution of the reaction mixture with water or a mixture of water with an organic solvent selected from the group of lower aliphatic alcohols or ketones;
h) isolation of a precipitated imatinib base; and
i) optionally, conversion of imatinib base by a known method into its pharmaceutically acceptable acid addition salt, preferably into a methanesulfonic acid salt.

Another aspect of the disclosure is a process for preparation of an intermediate in the imatinib synthesis, i.e. N-(5-nitro-2-methylphenyl)-4-(3-pyridinyl)-2-pyridine-amine, comprising:
a) preparation of a 1-(2-methyl-5-nitrophenyl)guanidine nitrate by reacting 2-methyl-5-nitroaniline with cyanamide in the presence of concentrated hydrochloric acid and conversion of the resulting hydrochloride acid salt into a nitric acid salt;
b) independently, condensation of 3-acetylpyridine with N,N-dimethylformamide dimethyl acetal under conditions favouring removal of methanol formed in the reaction, to obtain crude 3-(dimethylamino)-1-(3-pyridinyl)-prop-2-en-1-one, which, without further purification, is
c) reacted directly with 1-(2-methyl-5-nitrophenyl)-guanidine nitrate obtained in step a).

The other aspect of the disclosure is a process for preparation of an intermediate in the imatinib synthesis, i.e. N-(5-amino-2-methylphenyl)-4-(3-pyridinyl)-2-pyridine-amine by reduction of N-(5-nitro-2-methylphenyl)-4-(3-pyridinyl)-2-pyridineamine using hydrazine in the presence of Raney's nickel.

The further aspect of the disclosure is a process for preparation of an intermediate in the imatinib synthesis, i.e. 4-chloromethyl-N-{[4-methyl-3-(4-pyridin-3-yl)pyrimidin-2-ylamino]-phenyl}-benzamide comprising a condensation of N-(5-nitro-2-methylphenyl)-4-(3-pyridinyl)-2-pyridineamine with 4-chloromethylbenzoyl chloride in the presence of a base.

Yet another embodiment of the disclosure comprises a process for preparation of imatinib base by condensing 4-chloromethyl-N-{[4-methyl-3-(4-pyridin-3-yl)pyrimidin-2-ylamino]-phenyl}-benzamide with N-methylpiperazine, wherein the reaction is carried out in a molar excess of N-methylpiperazine that is also a solvent of the reaction, followed by diluting the reaction mixture with water or a mixture of water with an organic solvent selected from the group of lower aliphatic alcohols or ketones, and, if needed, neutralizing the reaction medium with an aqueous solution of an inorganic base, and finally, isolating imatinib base.

### Detailed description of the invention

The present invention provides an improved process for the preparation of imatinib base that ensures high yields of subsequent steps of the synthesis and simple, in terms of technology, methods for isolating and purifying intermediates thereof. The synthetic pathway is outlined on Scheme 4 below.

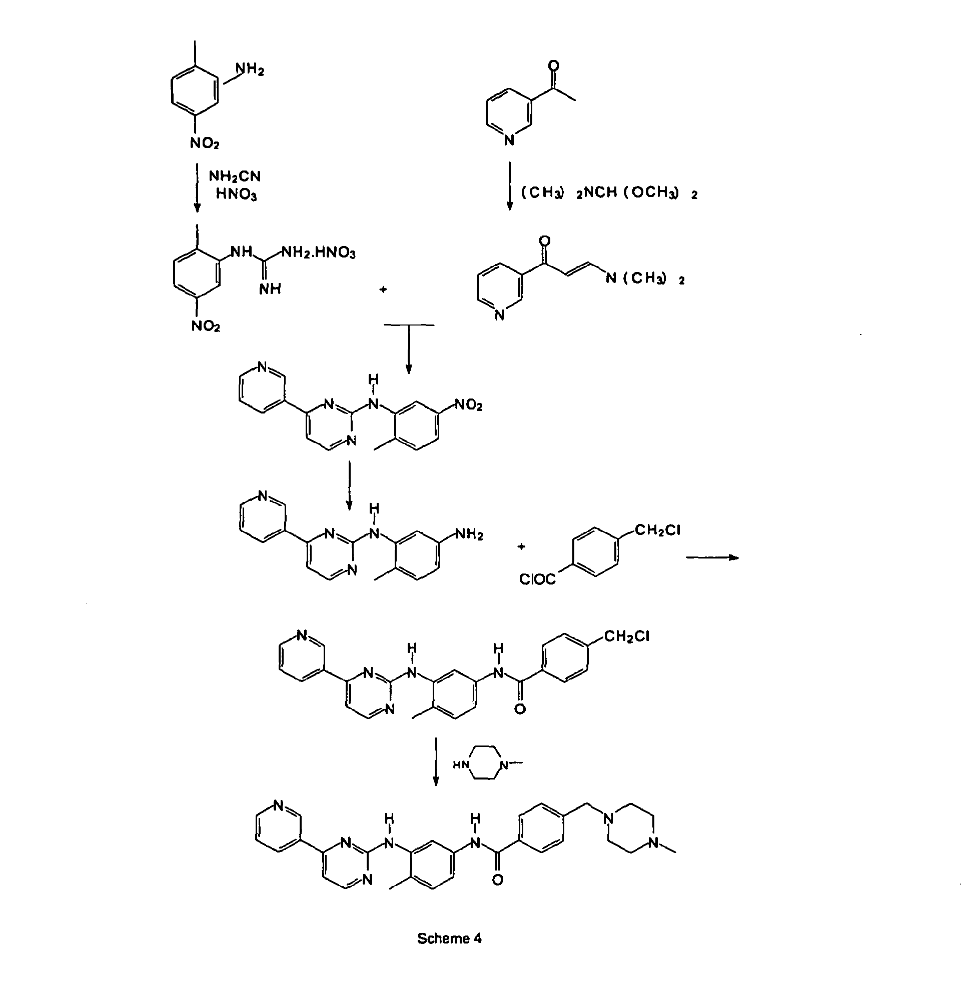

Successive steps of the process according to the invention are carried out in the following way. I. N-(5-Nitro-2-methylphenyl)-4-(3-pyridinyl)-2-pyrimidineamine.

N-(5-Nitro-2-methylphenyl)-4-(3-pyridinyl)-2-pyrimidineamine is obtained by condensation of 3-acetylpyridine with N,N-dimethylformamide dimethyl acetal under conditions favourable for removal methanol formed in the reaction.

The condensation of 3-acetylpyridine with N,N-dimethylformamide dimethyl acetal is carried out using a slight molar excess, e.g. 1.2-2.0, preferably 1.5 equivalent of the acetal per 1 equivalent of 3-acetylpyridine.

N,N-Dimethylformamide dimethyl acetal provides a suitable medium for the reaction, although it could be also carried out in a neutral organic solvent, the boiling temperature of which is not lower than that of the acetal used in the reaction.

The condensation is carried out at the boiling point of the reaction mixture until no 3-acetylpyridine is visible on a TLC. The reaction time in the case of using N,N-dimethylformamide dimethyl acetal is in general shorter compared to that in the prior art solutions.

Methanol, formed in the reaction can be removed from the reaction mixture by any method, e.g. by distillation, in a continuous way or periodically (in certain time intervals during the reaction or on a one-off basis after its completion) using an apparatus provided with a Vigreux distillation head.

The crude reaction product is obtained by distilling off the excess of the acetal and the additional organic solvent, if any. This product, without further purification is used in the next step of the synthesis by dissolving it in a suitable neutral solvent, preferably N,N-dimethylformamide, and adding a guanidine derivative nitrate and a solution of a base, if necessary.

The bases used in this reaction could be salts of alkaline metals and alkaline earth metals, such as carbonates and bicarbonates or hydroxides of alkaline metals, such as sodium or potassium hydroxide.

After completion of the reaction, the crude product is isolated in a standard way, e.g. by filtration and then it is dried and further purified by known methods, if necessary. In general, the product obtained by the process according to the invention does not require additional purification and can be used in the next step of the synthesis.

Crude 3-(dimethylamino)-1-(3-pyridinyl)-prop-2-en-1-one is reacted with 1-(2-methyl-5-nitrophenyl)guanidine nitrate without any purification.

The starting material, 1-(2-methyl-5-nitrophenyl)guanidine nitrate is obtained independently in a more efficient way compared to the prior art process, by reacting 2-methyl-5-nitroaniline with cyanamide in the presence of concentrated hydrochloric acid and then converting the guanidine hydrochloride derivative into a nitrate by treating it with concentrated nitric acid.

The yield of N-(5-nitro-2-methylphenyl)-4-(3-pyridinyl)-2-pyridineamine is over 73%, calculating on 3-acetylpyridine.

### II. N-(5-Amino-2-methylphenyl)-4-(3-pyridinyl)-2-pyrimidine-amine

In the next step, N-(5-nitro-2-methylphenyl)-4-(3-pyridinyl)-2-pyrimidineamine is reduced to N-(5-amino-2-methylphenyl)-4-(3-pyridinyl)-2-pyridineamine using hydrazine in the presence of Raney's nickel.

It has been found that certain conditions of carrying out the process reduce to minimum amounts of by-products formed in the reaction and facilitate isolation and purification of the product.

In certain circumstances, as for example, insufficient activity of Raney's nickel (e.g., in the case of using recovered Raney's nickel), too low amount of the catalyst, too low the addition rate of hydrazine, or if the crude nitro compound is wet, undesired reduction by-products are formed in the reaction. Removal of the impurities during purification of crude N-(5-amino-2-methylphenyl)-4-(3-pyridinyl)-2-pyridineamine is difficult, as these compounds are crystallizing much more readily than the major product of the reaction. Moreover, it was found that in case hydrazine in the presence of Raney's nickel is used as a catalytic system, these impurities are not reduced to amine. Practically, it makes their removal by a chemical treatment impossible.

Structure of each of the impurities has been determined by proton magnetic resonance, ¹H-NMR, and by combustion analysis. The structure la corresponds to the azo-derivative and the structure 1b to azoxy-derivative, represented by formulae below.

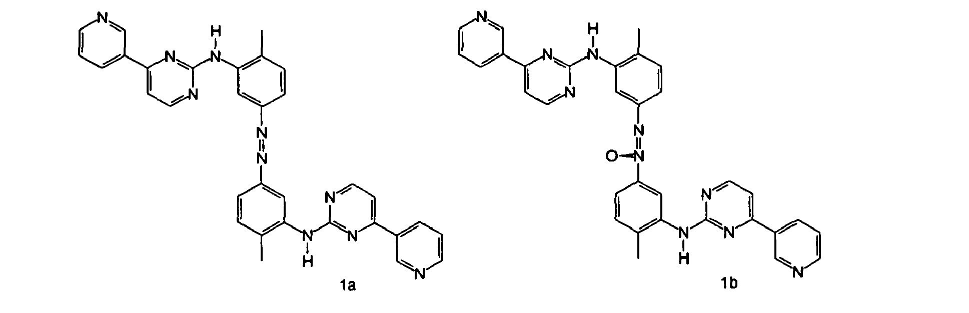

In a preferred embodiment of the invention, the reduction of N-(5-nitro-2-methylphenyl)-4-(3-pyridinyl)-2-pyridineamine to N-(5-amino-2-methylphenyl)-4-(3-pyridinyl)-2-pyridineamine is accomplished by using commercially available hydrazine hydrate or aqueous solutions thereof, e.g. a 40% or 80% solution.

A factor, that is crucial for decreasing the level of by-products, is to carry out the process while maintaining a permanent excess of hydrazine in the reaction medium. Usually, about 2 to 8 moles, preferably, about 3 to 4.5 moles of hydrazine hydrate is used per 1 mole of N-(5-nitro-2-methylphenyl)-4-(3-pyridinyl)-2-pyridineamine. Hydrazine hydrate is added to the reaction mixture in one portion, or in two or more portions.

In order to ensure a proper course of the reaction is to use not less than approximately 10% by weight of Raney's nickel accounting for dry N-(5-nitro-2-methylphenyl)-4-(3-pyridinyl)-2-pyridineamine.

Favourable reduction conditions are provided by using Raney's nickel of a high activity.

The reaction is carried out in an aliphatic alcohol, such as methanol, isopropanol, 2-methoxyethanol or in an aliphatic or cyclic ether such as tetrahydrofuran, dioxane or 1,2-dimethoxyethane. Preferably, the reduction is carried out in methanol.

The process is carried out by adding dropwise hydrazine hydrate to a suspension of the nitro compound at temperature ranging from room temperature to the boiling point of the reaction mixture until no starting material is detectable by TLC and then isolating the product by a typical work-up.

Hydrazine, used for the reduction, allows the product to be easily isolated and purified. The yield of the product exceeds 80% and its purity - 99.5% (by HPLC). The use of inexpensive and readily available reagents allows the process according to the invention to be scaled up to the full industrial scale.

### III. 4-Chloromethyl-N-{[4-methyl-3-(4-pyridin-3-yl)pyrimidin-2-ylamino]-phenyl}-benzamide.

4-Methyl-N-3-[(4-pyridin-3-yl)pyrimidin-2-yl)]-benzene-1,3-diamine is then reacted with 4-chloromethylbenzoyl chloride in the presence of a base.

Unlike 4-(4-methylpiperazinemethyl)benzoyl chloride used in the prior art solutions, in the present invention a readily available 4-chloromethylbenzoyl chloride can be used in the free form (not hydrochloride form).

The reaction is carried out in a neutral organic solvent such as tetrahydrofuran, dioxane or dimethylsulfoxide.

The base is used in the amount sufficient for binding the theoretical amount of hydrogen chloride present in the reaction medium. A suitable base could be any organic base, such as tertiary amines, e.g., triethylamine, or inorganic bases, such as carbonates or bicarbonates salts of alkaline metals, e.g., potassium carbonate, sodium carbonate or sodium bicarbonate. Potassium carbonate is particularly preferred. Next, the reaction mixture is diluted with water and the precipitated product is isolated by filtration.

Usually, the yield of this step of the synthesis exceeds 95%.

### IV. 4-(4-Methyl-piperazin-1-ylmethyl)-N-{(4-methyl-3-(4-pyridin-3-yl)pyrimidin-2-ylamino]-phenyl}-benzamide.

4-Chloromethyl-N-{[4-methyl-3-(4-pyridin-3-yl)pyrimidin-2-ylamino]-phenyl}-benzamide obtained in the previous step is reacted with N-methylpiperazine. The reaction is carried out using a molar excess of the latter reagent playing also a role of a solvent, or in its mixture with a neutral organic solvent, e.g. a cyclic ether.

The molar excess of N-methylpiperazine is from about 2 to about 12, preferably from about 5 to about 7 equivalents with respect to 4-chloromethyl-N-{[4-methyl-3-(4-pyridin-3-yl)pyrimidin-2-ylamino]-phenyl}-benzamide. In general, the reaction is carried out at temperature 120-140°C for 1.5-3 hours. After completion of the reaction, water or mixture of water and an organic solvent, selected from lower aliphatic alcohols or ketones, is added to the reaction mixture and the reaction medium is neutralized, if necessary, with an aqueous solution of an alkaline metal hydroxide or bicarbonate. The precipitate is isolated, e.g., by filtration and dried in air to afford a crystalline product of high chromatographic purity with the yield over 90%. Conventional purification, e.g., single recrystallization is sufficient for obtaining a product complying with pharmacopoeial purity requirements.

The improved steps of the process according to the invention provide an improved and efficient process for preparation of imatinib base. The process allows for using simple starting materials and reagents, while simultaneously avoiding a laborious isolation and purification of intermediates and final product, therefore facilitating its further development to the industrial scale.

A total yield of imatinib base is over 45%, compared to approximately 15% reported in the prior art processes.

The following examples are provided to illustrate the invention. The examples are not meant to limit the scope of the invention as defined in the claims.

### Examples

### 1-(2-Methyl-5-nitrophenyl)guanidine nitrate

### Example 1

20g of 2-Methyl-5-nitroaniline (0.131 M; 1 eq.), 11.05g of cyanamide (0.263 M; 2 eq.) and 80 mL of isopropyl alcohol were placed in a reactor flask. The reaction mixture was heated to 80°C and 18 mL of concentrated hydrochloric acid was slowly added dropwise within 80 minutes. The reaction mixture was stirred for 1 hour while maintaining it at 80°C. Next, 6 mL of concentrated hydrochloric acid was added dropwise and the reaction mixture was kept at 80°C for 2 hours. The reaction mixture was cooled down to 60°C and 10 mL of 65% nitric acid (0.142 M, 1.08 eq.) was added dropwise and the mixture was left with stirring until it was cooled down to room temperature. The solid product was filtered off, washed with 50 mL of isopropyl alcohol and dried in the air to afford 27.45 g (yield 81.1%) of 1-(2-methyl-5-nitrophenyl)guanidine nitrate, m.p.203-206°C;
¹H NMR (DMSO-d₆, 200 MHz): 2.32 (3H, s, CH₃), 7.44 (4H, m, 4 x NH), 7.64 (1H, d, J=8.2 Hz, 3-H), 8.09 (1H, d, J=2.5 Hz, 6-H), 8.15 (1H, dd, J=8.2 i 2.5 Hz, 4-H), 9.51 (1H, m, NH⁺).

### N-(5-Nitro-2-methylphenyl)-4-(3-pyridinyl)-2-pyrimidineamine.

### Example 2

26.60 g of 3-acetylpyridine (0.219 M; 1 eq.) and 44.0 mL of N,N-dimethylformamide dimethyl acetal (39.47 g; 0.331 M; 1.508 eq.) were placed in a reactor flask and the mixture was refluxed for 1.5 h. Methanol, produced in the reaction was removed by distillation and the mixture was refluxed for further 2 hours. The rest of methanol and excess of the acetal were removed by distillation under slightly reduced pressure. The hot residue was treated with 100 mL of DMF, and, after cooling down, with 56.3 g of 1-(2-methyl-5-nitrophenyl)guanidine nitrate (0.219 M; 1 eq.). A solution of 8.8 g of sodium hydroxide in 17 mL of water was then added dropwise and the mixture was refluxed for 8 hours in an oil bath. Water (200 mL) was added with care to the hot solution, and the content of the flask was poured to 700 mL of warm water. The whole mixture was cooled with stirring to room temperature. The precipitated solid was isolated by filtration and dried in the air to afford 49.41g of crude 2-(2-methyl-5-nitroanilino)-4-(3-pyridinyl)pyrimidine (yield 73.2% calculated on 3-acetylpyridine), m.p. 186-188°C; ¹H NMR (DMSO-d₆, 200 MHz): 2.43 (3H, s, CH₃), 7.54 (3H, m, 3-H phenyl+ 5-H pyrimidine + 5-H pyridil), 7.90 (1H, dd, J=8.2 i 2.2 Hz, 4-H phenyl), 8.48 (1H, dt, J₁=7.9 Hz, 4-H pyridil), 8.62 (1H, d, J=5.4 Hz, 6-H pyrimidine), 8.71 (1H, dd, 6-H pyridil), 8.79 (1H, d, J=2.2 Hz, 6-H phenyl), 9.27 (1H, s, NH), 9.32 (1H, d, J=1.6 Hz, 2-H pyridil).

### N-(5-Amino-2-methylphenyl)-4-(3-pyridinyl)-2-pyrimidineamine

### Example 3

To a stirred suspension of N-(5-nitro-2-methylphenyl)-4-(3-pyridinyl)-2-pyridineamine (65 g; 0.21 M) in methanol (1400 mL) was added wet Raney's nickel (6.25 g) followed by the first portion of an 80% solution of hydrazine hydrate (52.5 g; 0.84 M) in methanol (50 mL). After 1 hour, the reaction mixture was cooled down to 35°C and stirring was continued for further 1.5 hour. Next, the second portion of an 80% solution of hydrazine hydrate (52.5 g; 0.84 M) in methanol (50 mL) was added dropwise and stirring was continued for 3 hours. The reaction mixture was cooled down to room temperature and filtered through a pad of Celite. The layer of Celite was washed with methanol (150 mL). The combined filtrates were concentrated *in vacuo*, treated with dichloromethane (500 mL), and the organic layer was washed with water (3 x 300 mL). The organic layer was dried with anhydrous MgSO₄ (12 g) and filtered through a pad of SiO₂ (60 g). The layer of silica gel was washed subsequently with a mixture of dichloromethane-ethyl acetate (50:50; 300 mL) and ethyl acetate (600 mL). The combined filtrates were concentrated under normal pressure to approximately 100 mL and cooled down with stirring to room temperature. The crystalline solid was filtered off and washed with a small volume of dichloromethane to afford 47.5 g (80.9 %) of the product. Purity (by HPLC): 99.84%.

### Example 4

Wet Raney's nickel (2.5 g) was added to a stirred suspension of N-(5-nitro-2-methylphenyl)-4-(3-pyridinyl)-2-pyridineamine (24 g; 78.1 mM) in methanol (380 mL). The mixture was heated to reflux and the first portion of a 40% solution of hydrazine hydrate (10 mL; 79.9 mM) was added dropwise. After refluxing the mixture for 20 minutes, the second portion of a 40% solution of hydrazine hydrate (10 mL; 79.9 mM) was added dropwise. After 40 minutes of refluxing, the reaction mixture was cooled down to room temperature and filtered through a pad of Celite. The layer of Celite was washed with methanol (150 mL). The combined filtrates were concentrated *in vacuo.* The residue was treated with dichloromethane (300 mL) and active charcoal (2.5 g) and then refluxed for 30 minutes. After cooling the reaction mixture to room temperature, charcoal was filtered off and washed with dichloromethane (100 mL). Next, the procedure described in Example 3 was followed to afford 16.552 g (76.4%) of the product. Purity (by HPLC): 99.64%.

### Example 5

Wet Raney's nickel (2.5 g) was added to a stirred suspension of N-(5-nitro-2-methylphenyl)-4-(3-pyridinyl)-2-pyridineamine (24 g; 78.1 mM) in methanol (380 mL). The mixture was heated to reflux and the first portion of an 80% solution of hydrazine hydrate (7.5 mL; 119.9 mM) was added dropwise. After refluxing the mixture for 20 minutes, the second portion of an 80% solution of hydrazine hydrate (7.5 mL; 119.9 mM) was added dropwise. After 40 minutes of refluxing, the reaction mixture was cooled down to room temperature and filtered through a pad of Celite. The layer of Celite was washed with methanol (100 mL). The combined filtrates were concentrated *in vacuo*. The residue was treated with dichloromethane (200 mL) and active charcoal (2.5 g) and then refluxed for 30 minutes. After cooling the reaction mixture to room temperature, charcoal was filtered off and washed with dichloromethane (100 mL). Next, the procedure described in Example 3 was followed to afford 15.886 g (73.2%) of the product. Purity (by HPLC): 99.75%.

### 4-Chloromethyl-N-[4-methyl-3-(4-pyridin-3-yl-pyrimidin-2-ylamino)-phenyl]-benzamide.

### Example 6

4-Methyl-N-3-(4-pyridin-3-ylpyrimidin-2-yl)-benzene-1,3-diamine (18.488 g, 0.067 M) was dissolved in THF (255 mL), freshly ground potassium carbonate (19.870 g, 0.143 M) was added and then the mixture was cooled down to 0°C. A solution of 4-chloromethylbenzoyl chloride (13.863 g, 0.073 M) in THF (69 mL) was added dropwise within 15 minutes while temperature of the reaction mixture was maintained at 0°C. The reaction mixture was stirred at this temperature for 2 hours and for another 2 hours at room temperature. Water was added dropwise at a very low rate. Temperature of the reaction has risen to 26°C. At this temperature dropwise addition of water was continued with cooling. Cooling was discontinued when temperature dropped below 19°C. When water addition was completed (in total 543 mL), temperature of the reaction mixture was 26°C. The reaction mixture was stirred for further 30 min. A solid precipitate was filtered off and washed with water (150 mL) to afford 28.06 g (yield 97%) of the title compound in the form of a colourless crystalline solid.
M.p. 211-212°C. ¹H NMR (DMSO-d₆): 10.24 (1H, s), 9.28 (1H, d, J=1,8), 8.99 (1H, s), 8.69 (1H, dd, J=4.8-1.4), 8.52 (1H, d, J=5,2), 8.48 (1H, dt, J=8.2-1.8), 8.09 (1H, d, J=2.0), 7.96 (2H, d, J=8.0), 7.51 (5H, m), 7.22 (1H, d, J=8.4), 4.85 (2H, s), 2.23 (3H, s).

### Example 7

4-Methyl-N-3-[(4-pyridin-3-yl)pyrimidin-2-yl]-benzene-1,3-diamine (18.488 g, 0.067 M) was dissolved in THF (255 mL), freshly ground potassium carbonate (19.870 g, 0.143 M) was added and then the mixture was cooled down to 0°C. A solution of 4-chloromethylbenzoyl chloride (13.863 g, 0.073 M) in THF (69 mL) was added dropwise within 10 minutes while temperature of the reaction mixture was maintained at approximately 20°C. The reaction mixture was stirred at room temperature for 1 hour. Water was added dropwise (in total 543 mL) while maintaining temperature of the reaction mixture at approximately 20°C, and then the reaction mixture was stirred for 80 min. A solid precipitate was filtered off and washed with water (150 mL) to afford 28.37 g (yield 98%) of the title compound.

### Example 8

4-Methyl-N-3-[(4-pyridin-3-yl)pyrimidin-2-yl]-benzene-1,3-diamine (9.244 g, 33.3 mM) was dissolved in THF (128 mL), then a solution of potassium carbonate (9.935 g, 71.9 mM) in water (13 mL) was poured in. The mixture was cooled down to 0°C and a solution of 4-chloromethylbenzoyl chloride (6.932 g, 36.7 mM) in THF (30 mL) was added dropwise within 15 minutes. The reaction mixture was stirred at 0°C for 2 hours. Water (260 mL) was added dropwise with stirring and cooling the reaction flask and then at room temperature for further 30 min. A solid precipitate was filtered off and washed with water (120 mL) to afford 13.62 g (yield 95%) of the title compound.

### 4-(4-Methyl-piperazin-1-ylmethyl)-N-{4-methyl-3-[(4-pyridin-3-yl)pyrimidin-2-ylamino]-phenyl}-benzamide

### Example 9

A flask containing a mixture of 4-chloromethyl-N-{4-methyl-3-[(4-pyridin-3-yl)pyrimidin-2-ylamino]-phenyl}-benzamide obtained in the previous step (56.072 g; 0.130 M), N-methylpiperazine (84 mL; 75.852 g; 0.757 M) and dioxane (56 mL) was placed in an oil bath at temperature 135°C and the reaction mixture was heated for 3 hours. After that time the oil bath was taken away and after cooling the reaction mixture, acetone (150 mL) was added and stirring was continued until the mixture was cooled down to room temperature. The precipitated product was filtered off, washed with cold acetone (50 mL) and dried in the air to afford 59.2 g (yield 91%) of the title compound in the form of a cream-coloured, crystalline solid. M.p. 167-170°C. ¹H NMR (DMSO-d₆): 10.16 (1H, s), 9.27 (1H, d, J=2.2), 8.98 (1H, s), 8.68 (1H, dd, J=4.8-1.6), 8.51 (1H, d, J=5.3), 8.48 (1H, dt, J=8.2-2.0), 8.08 (1H, d, J=1.8), 7.90 (2H, d, J=8.2), 7.45 (5H, m), 7.20 (1H, d, J=8.6), 3.52 (2H, s), 2.35 (8H, m), 2.22 (3H, s), 2.15 (3H,s).

### Example 10

A flask containing a mixture of 4-chloromethyl-N-{4-methyl-3-[(4-pyridin-3-yl)pyrimidin-2-ylamino]-phenyl}-benzamide (55.276 g; 0.129 M) and N-methylpiperazine (83 mL; 74.949 g; 0.748 M) was placed in an oil bath at temperature 120-125°C and the reaction mixture was heated for 90 minutes. After that time the oil bath was taken away, isopropanol (300 mL) was added to the hot mixture and stirring was continued until the mixture was cooled down to room temperature. The precipitated product was filtered off, washed with cold isopropanol (50 mL) and dried in the air to afford 58.7 g (92%) of the product.

### Example 11

A flask containing a mixture of 4-chloromethyl-N-{4-methyl-3-[(4-pyridin-3-yl)pyrimidin-2-ylamino]-phenyl}-benzamide (54.953 g; 0.129 M) and N-methylpiperazine (83 mL; 74.949 g; 0.748 M) was placed in an oil bath at temperature 120-125°C and the reaction mixture was heated for 90 minutes. After that time the oil bath was taken away, isopropanol (200 mL) was added to the hot mixture and stirring was continued until the mixture was cooled down to room temperature. The precipitated product was filtered off, washed with a mixture of isopropanol and water (1:1, 100 mL) and dried in the air to afford 57.7g (91%) of the product.

### Example 12

A flask containing a mixture of 4-chloromethyl-N-{4-methyl-3-[(4-pyridin-3-yl)pyrimidin-2-ylamino]-phenyl}-benzamide (54.949g; 0.129 M) and N-methylpiperazine (83 mL; 74.949 g; 0.748 M) was placed in an oil bath at temperature 120-125°C and the reaction mixture was heated for 90 minutes. After that time the oil bath was taken away, water (100 mL) was added to the hot mixture and stirring was continued until the mixture was cooled down to room temperature. The precipitated product was filtered off and the residue was taken from the flask with water (80 mL). The whole precipitate was washed with water (3×100 mL) to afford 60.2g (95%) of the product.

### Example 13

A flask containing a mixture of 4-chloromethyl-N-{4-methyl-3-[(4-pyridin-3-yl)pyrimidin-2-ylamino]-phenyl}-benzamide (55.000g; 0.128 M) and N-methylpiperazine (83 mL; 74.949 g; 0.748 M) was placed in a cold oil bath and the reaction mixture was heated until the bath temperature has reached 130°C. Heating at that temperature was continued for 90 minutes. After that time the oil bath was taken away, and when temperature of the reaction mixture dropped to 95°C, water (100 mL) was added. Stirring was continued until the mixture was cooled down to room temperature. A solution of sodium hydroxide (5.12g) in water (100 mL) was added and the mixture was stirred for 30 min. The precipitated product was filtered off, washed with water (300 mL) to afford 60.6g (96%) of the product.

## Claims

1. A process for preparation of imatinib base and pharmaceutically acceptable acid addition salts thereof, comprising:
a) reacting 2-methyl-5-nitroaniline with cyanamide in the presence of concentrated hydrochloric acid to obtain 1-(2-methyl-5-nitrophenyl)-guanidine hydrochloride and converting said 1-(2-methyl-5-nitrophenyl)-guanidine hydrochloride into 1-(2-methyl-5-nitrophenyl)-guanidine nitrate;
b) independently, condensing 3-acetylpyridine with N,N-dimethylformamide dimethyl acetal using a molar excess of 1.2-2.0 equivalents of the said acetal per 1 mole of 3-acetylpyridine and while removing methanol in course of the reaction by distillation, to obtain 3-(dimethylamino)-1-(3-pyridinyl)-prop-2-en-1-one;
c) reacting 3-(dimethylamino)-1-(3-pyridinyl)-prop-2-en-1-one obtained in step b) without its purification with 1-(2-methyl-5-nitrophenyl)-guanidine nitrate obtained in step a) to obtain N-(5-nitro-2-methylphenyl)-4-(3-pyridinyl)-2-pyridineamine;
d) reducing N-(5-nitro-2-methylphenyl)-4-(3-pyridinyl)-2-pyridineamine obtained in the step c) to N-(5-amino-2-methylphenyl)-4-(3-pyridinyl)-2-pyridineamine using hydrazine in the presence of Raney's nickel, wherein hydrazine is present in the reaction medium in the permanent excess;
e) condensing N-(5-amino-2-methylphenyl)-4-(3-pyridinyl)-2-pyridineamine obtained in step d) with 4-chloromethylbenzoyl chloride in the presence of an inorganic base, diluting the reaction mixture with water and isolating the precipitated 4-chloromethyl-N-{[4-methyl-3-(4-pyridin-3-yl)pyrimidin-2-ylamino]-phenyl}-benzamide;
f) condensing 4-chloromethyl-N-{[4-methyl-3-(4-pyridin-3-yl)pyrimidin-2-ylamino]-phenyl}-benzamide obtained in step e) with N-methylpiperazine, wherein N-methylpiperazine is used in a molar excess of 2-12 equivalents with respect to 4-chloromethyl-N-{[4-methyl-3-(4-pyridin-3-yl)pyrimidin-2-ylamino]-phenyl}-benzamide;
g) diluting the reaction mixture with water or isopropanol or acetone, and optionally neutralizing;
h) isolating imatinib base precipitated in step g); and
i) optionally, converting imatinib base by a known method into its pharmaceutically acceptable acid addition salt.

2. The process according to Claim 1, wherein condensation of 3-acetylpyridine with N,N-dimethylformamide dimethyl acetal is carried using a molar excess of 1.5 equivalent of the acetal per 1 mole of 3-acetylopyridine.

3. The process according to Claim 1, wherein the reduction of N-(5-nitro-2-methylphenyl)-4-(3-pyridinyl)-2-pyrimidineamine to N-(5-amino-2-methylphenyl)-4-(3-pyridinyl)-2-pyrimidine-amine in step d) is carried out in the presence of hydrazine hydrate or aqueous solutions thereof.

4. The process according to Claim 1, wherein the amount of hydrazine hydrate used in the reduction in step d) is from about 2 to 8, preferably from 3 to 4.5 moles per 1 mole of N-(5-nitro-2-methylphenyl)-4-(3-pyridinyl)-2-pyrimidineamine.

5. The process according to Claim 1, wherein Raney's nickel is used in the reduction in step d) in the amount not less than approximately 10% by weight with respect to N-(5-nitro-2-methylphenyl)-4-(3-pyridinyl)-2-pyrimidineamine.

6. The process according to Claim 1, wherein reduction in step d) is carried out using an alcohol or aliphatic or cyclic ether as a solvent.

7. The process according to Claim 6, wherein reduction in step d) is carried out in methanol.

8. The process according to Claim 1, wherein N-(5-amino-2-methylphenyl)-4-(3-pyridinyl)-2-pyrimidine-amine obtained in step d) is free of azo- and azoxy-derivative.

9. The process according to Claim 1, wherein the reaction of 4-methyl-N-3-[(4-pyridin-3-yl)pyrimidin-2-yl]-benzene-1,3-diamine with 4-chloromethylbenzoyl chloride is carried out in the presence of potassium carbonate.

10. The process according to Claim 1, wherein in step f) an excess of 5-7 equivalents of N-methylpiperazine is used with respect to 4-chloromethyl-N-{[4-methyl-3-(4-pyridin-3-yl)pyrimidin-2-ylamino]-phenyl}-benzamide.

## Patentansprüche

1. Verfahren zur Herstellung von Imatinib-Base und ihren pharmazeutisch verträglichen Additionssalzen mit Säuren, umfassend:
a) Umsetzung von 2-Methyl-5-nitroanilin mit Cyanamid in Gegenwart von konzentrierter Salzsäure um 1-(2-Methyl-5-nitrophenyl)-guanidin-hydrochlorid zu erhalten und Umsetzung von dem obengenannten 1-(2-Methyl-5-nitrophenyl)-guanidin-hydrochlorid in 1-(2-Methyl-5-nitrophenyl)-guanidin-nitrat;
b) unabhängig davon, Kondensation von 3-Acetylpyridin mit N,N-Dimethylformamid-dimethylacetal unter Verwendung von molarem Überschuß von 1.2-2.0 Äquivalenten des genannten Acetals per 1 Mol 3-Acetylpyridin und Abdestillieren von Methanol während der Reaktion, um 3-(Dimethylamino)-1-(3-pyridinyl)-prop-2-en-1-on zu herstellen;
c) Umsetzung von in Schritt b hergestellten nicht aufreinigten 3-(Dimethylamino)-1-(3-pyridinyl)-prop-2-en-1-ons mit dem in Schritt a) hergestellten 1-(2-Methyl-5-nitrophenyl)-guanidin-nitrat, um N-(5-Nitro-2-methylphenyl)-4-(3-pyridinyl)-2-pyridinamin zu herstellen;
d) Reduktion von dem in Schritt c) hergestellten N-(5-Nitro-2-methylphenyl)-4-(3-pyridinyl)-2-pyridinamin, mit Verwendung von Hydrazin in Gegenwart von Raney-Nickel, um N-(5-Amino-2-methylphenyl)-4-(3-pyridinyl)-2-Pyridinamin zu herstellen, wobei Hydrazin im Reaktionsmedium in permanentem Überschuss vorliegt;
e) Kondensation von dem in Schritt d) hergestellten N-(5-Amino-2-methylphenyl)-4-(3-pyridinyl)-2-pyridinamin mit 4-Chlormethylbenzoylchlorid in Gegenwart von anorganischer Base, Verdünnen der Reaktionsmischung mit Wasser und Isolieren des ausgefallenen 4-Chloromethyl-N-{[4-methyl-3-(4-pyridin-3-yl)pyrimidin-2-ylamino]-phenyl}-benzamides;
f) Kondensation des in Schritt e) hergestellten 4-Chloromethyl-N-{[4-methyl-3-(4-pyridin-3-yl)pyrimidin-2-ylamino]-phenyl}-benzamides mit N-Methylpiperazin, wobei N-Methylpiperazin in molarem Überschuß von 2-12 Äquivalenten, bezogen auf 4-Chloromethyl-N-{[4-methyl-3-(4-pyridin-3-yl)pyrimidin-2-ylamino]-phenyl}-benzamid verwendet wird;
g) Verdünnen der Reaktionsmischung mit Wasser oder Isopropanol oder Aceton, und ggf. Neutralisieren;
h) Isolieren der in Schritt g) ausgefallenen Imatinib-base; und
i) ggf. Umsetzung von der Imatinib-base mit bekannter Methode in ihr pharmazeutisch verträglichen Additionssalz mit Säure.

2. Verfahren nach Anspruch 1, **dadurch gekennzeichnet dass** die Kondensation von 3-Acetylpyridin mit N,N-Dimethylformamid-dimethyl-acetal unter Verwendung von molarem Überschuss von 1.5 Äquivalenten Acetal per 1 Mol 3-Acetylpyridin durchgefuhrt wird.

3. Verfahren nach Anspruch 1, **dadurch gekennzeichnet dass** die Reduktion von N-(5-Nitro-2-methylphenyl)-4-(3-pyridinyl)-2-pyrimidinamin zu N-(5-Amino-2-methylphenyl)-4-(3-pyridinyl)-2-pyrimidin-amin in Schritt d) in Gegenwart von Hydrazin-hydrat oder ihren Wasserlösungen durchgeführt wird.

4. Verfahren nach Anspruch 1, **dadurch gekennzeichnet dass** die in der Reduktion in Schritt d) verwendete Menge von Hydrazin-hydrat von etwa 2 bis 8, bevorzugt von 3 bis 4,5 Mol, per 1 Mol N-(5-Nitro-2-methylphenyl)-4-(3-pyridinyl)-2-pyrimidinamin beträgt.

5. Verfahren nach Anspruch 1, **dadurch gekennzeichnet dass** die in der Reduktion in Schritt d) verwendete Menge von Raney-Nickel nicht weniger als ca. 10 Gew.-% bezogen auf N-(5-Nitro-2-methylphenyl)-4-(3-pyridinyl)-2-pyrimidinamin beträgt.

6. Verfahren nach Anspruch 1, **dadurch gekennzeichnet dass** die Reduktion in Schritt d) unter Verwendung von Alkohol oder aliphatischen oder zyklischen Ether als Lösungsmittel durchgeführt wird.

7. Verfahren nach Anspruch 6, **dadurch gekennzeichnet dass** die Reduktion in Schritt d) in Methanol durchgeführt wird.

8. Verfahren nach Anspruch 1, **dadurch gekennzeichnet dass** das in Schritt d) hergestellte N-(5-Amino-2-methylphenyl)-4-(3-pyridinyl)-2-pyrimidin-amin Azo- und Azoxyderivatfrei ist.

9. Verfahren nach Anspruch 1, **dadurch gekennzeichnet dass** die Umsetzung von 4-Methyl-N-3-[(4-pyridin-3-yl)pyrimidin-2-yl]-benzen-1,3-diamin mit 4-Chloromethylbenzoyl-chlorid in Gegenwart von Kaliumcarbonat durchgeführt wird.

10. Verfahren nach Anspruch 1, **dadurch gekennzeichnet dass** in Schritt f) ein Überschuß von 5-7 Äquivalenten von N-Methylpiperazin bezogen auf 4-Chloromethyl-N-{[4-methyl-3-(4-pyridin-3-yl)pyrimidin-2-ylamino]-phenyl}-benzamid verwendet wird.

## Revendications

1. Procédé de fabrication de base d'imatinib et leur sels avec acides pharmaceutiquement acceptables, comportant:
a) réaction de 2-méthyl-5-nitroaniline avec cyanamide en présence d'acide chlorhydrique concentré pour obtenir chlorhydrate de 1-(2-méthyl-5-nitrophényl)-guanidine et conversion dudit chlorhydrate de 1-(2-méthyl-5-nitrophényl)-guanidine en nitrate de 1-(2-méthyl-5-nitrophényl)-guanidine;
b) independentement, condensation de 3-acétylpyridine avec diméthyl-acétal de N,N-diméthylformamide avec un excès molaire de 1.2-2.0 équivalents dudit acétal per 1 mole de 3-acétylpyridine pour conduire à 3-(diméthylamino)-1-(3-pyridinyl)-prop-2-ène-1-one en éliminant méthanol par distillation pendant la réaction;
c) réaction de 3-(diméthylamino)-1-(3-pyridinyl)-prop-2-ène-1-one obtenu en étape b) non purifié avec nitrate de 1-(2-méthyl-5-nitrophényl)-guanidine obtenu en étape a) pour conduire à N-(5-nitro-2-méthylphényl)-4-(3-pyridinyl)-2-pyridine-amine;
d) réduction de N-(5-nitro-2-méthylphényl)-4-(3-pyridinyl)-2-pyridine-amine obtenu en étape c) en N-(5-amino-2-méthylphényl)-4-(3-pyridinyl)-2-pyridine-amine en utilisant hydrazine en présence de nickel de Raney, hydrazine étant présent en milieu de réaction en excès permanent;
e) condensation de N-(5-amino-2-méthylphényl)-4-(3-pyridinyl)-2-pyridine-amine obtenu en étape d) avec chlorure de 4-chlorométhylbenzoyle en présence de base anorganique, dilution de mélange de réaction avec eau et isolation de 4-chlorométhyl-N-{[4-méthyl-3-(4-pyridin-3-yl)pyrimidin-2-ylamino]-phényl}-benzamide precipité;
f) condensation de 4-chlorométhyl-N-{[4-méthyl-3-(4-pyridin-3-yl)pyrimidin-2-ylamino]-phényl}-benzamide obtenu en étape e) avec N-méthylpiperazine, N-méthylpiperazine étant utilisé en excès molaire de 2-12 équivalents par rapport à 4-chlorométhyl-N-{[4-méthyl-3-(4-pyridin-3-yl)pyrimidin-2-ylamino]-phényl}-benzamide;
g) dilution de mélange de réaction avec eau ou isopropanol ou acétone, et éventuellement neutralisation;
h) isolation de base d'imatinib precipitée en étape g); et
i) éventuellement, conversion de base d'imatinib en utilisant la méthode connue en leur sel d'addition avec acide pharmaceutiquement acceptable.

2. Procédé selon revendication 1, **caracterisé en ce que** condensation de 3-acétylpyridine avec diméthyl-acétal de N,N-diméthylformamide est éxecuté en utilisant un excès molaire de 1.5 équivalents d'acétal par 1 mole de 3-acétylpyridine.

3. Procédé selon revendication 1, **caracterisé en ce que** la réduction de N-(5-nitro-2-méthylphényl)-4-(3-pyridinyl)-2-pyrimidine-amine en N-(5-amino-2-méthylphényl)-4-(3-pyridinyl)-2-pyrimidine-amine en étape d) est éxecuté en présence d'hydrate d'hydrazine ou leur solutions aqueuses.

4. Procédé selon revendication 1, **caracterisé en ce que** la quantité d'hydrate d'hydrazine utilisé pour la réduction en étape d) est de 2 environ à 8, de préférence de 3 à 4.5 moles par 1 mole de N-(5-nitro-2-méthylphényl)-4-(3-pyridinyl)-2-pyrimidine-amine.

5. Procédé selon revendication 1, **caracterisé en ce que** nickel de Raney est utilisé pour réduction en étape d) en quantité au moins de 10% en poids environ par rapport à N-(5-nitro-2-méthylphényl)-4-(3-pyridinyl)-2-pyrimidine-amine.

6. Procédé selon revendication 1, **caracterisé en ce que** la réduction en étape d) est éxecuté en utilisant alcool ou éther aliphatique ou cyclique comme un solvant.

7. Procédé selon revendication 6, **caracterisé en ce que** la réduction en étape d) est éxecuté en methanol.

8. Procédé selon revendication 1, **caracterisé en ce que** N-(5-amino-2-méthylphényl)-4-(3-pyridinyl)-2-pyrimidine-amine obtenu en étape d) est dépourvu de azo- et azoxy-derivés.

9. Procédé selon revendication 1, **caracterisé en ce que** la réaction de 4-méthyl-N-3-[(4-pyridin-3-yl)pyrimidin-2-yl]-benzène-1,3-diamine avec chlorure de 4-chlorométhylbenzoyle est éxecuté en présence de carbonate de potassium.

10. Procédé selon revendication 1, **caracterisé en ce que** en étape f) un excès de 5-7 équivalents de N-méthylpiperazine est utilisé par rapport à 4-chlorométhyl-N-{[4-méthyl-3-(4-pyridin-3-yl)pyrimidin-2-ylamino]-phényl}-benzamide.
